# EUROPEAN PATENT APPLICATION

(11) **EP 1 136 051 A1**
(43) Date of publication of application: **26.09.2001**
(21) Application number: 00106376.7
(22) Date of filing: 24.03.2000
(51) Int. Cl.: A61F 13/471

(54) **Male incontinence device**

(71) Applicant: THE PROCTER & GAMBLE COMPANY, Cincinnati, Ohio 45202 (US)
(72) Inventor: Schmitt, Achim, 55424 Münster-Sarmsheim (DE); Findeis, Bernd, 65843 Sulzbach/Ts. (DE); Blanco, Agustin Ramos, 61440 Oberursel (DE); Janki, Beate, , (DE)
(74) Representative: Kohol, Sonia

(57) **Abstract**

The present invention relates to an absorbent article, more particularly to an absorbent article to be worn by incontinent males. Claimed and described is an absorbent article being provided from a substantially rectangular sheet of material, the sheet of material comprising at least one line of juncture where the sheet is joined to itself as to form a pouch, characterised in that the sheet has a length to width ratio of at least 1.2:1. In another aspect of the present invention the absorbent article can be used in two different orientations.

## Description

### Field of the invention

The present invention relates to an absorbent article, more particularly to an absorbent article to be worn by incontinent males. In one preferred embodiment of the present invention an absorbent article is provided to tightly fit around the entire male genitalia.

### Background of the invention

A large variety of absorbent articles for incontinent people is found in the marketplace trying to address the needs in particular of the elderly and the unhealthy, but also of babies. Some of these people, typically for example babies, are both faecal and urine incontinent and may use products such as diapers to address their needs. Other people may be only urine incontinent or if both may see a need to address their urine incontinence by an individual product.

While urine incontinence of, for example, elderly women is a problem known to occur not infrequently and especially in case of light incontinence of women is often readily addressed by the use of sanitary napkins, urine incontinence of men, namely elderly men, so far has been less satisfactorily addressed despite deserving a similar attention in view of the ageing population in many countries.

Widely used are incontinence pads which may for additional protection and for better fit be worn in an incontinence garment. While these pads may provide sufficient storage capacity, they seem more appropriate for the female anatomy than for the male anatomy. Either such a pad is pressed so tightly against the body e.g. by an incontinence garment, that the wearing comfort is affected, or there is a risk of leakage - in particular since the penis and thereby the urinary duct has some freedom to move and hence urine may be deposited towards the edge or even outside the pad.

Other incontinence devices disclosed in the prior art comprise the following:

A number of so-called drop collectors is disclosed, these products provide limited absorbent capacity and are typically used in cases of light incontinence. WO 89/11839 discloses a drop collector to be worn around the penis. The disclosed product is not intended the cover the scrotum. WO 97/22316 discloses a similar device of a somewhat different shape. EP 0 903 131 discloses an incontinence guard comprising two pads suitable for introduction of the penis there-between. Portions of the pad material in use may lie adjacent to the scrotum.

Some other products while also designed for insertion of the penis only and not for coverage of the whole male genitalia appear to provide some but more absorbent capacity. For example US 5,695,485 discloses a pouch for insertion of the penis. EP 0 861 643 discloses an incontinence product comprising an urine absorbent bag for insertion of the penis. EP 0 776 642 and US 5,827,250 disclose urine absorbing bags comprising an opening for insertion of the wearer's penis. US 5,318,549 discloses an urine bag made of liquid impervious paper suitable for insertion of an absorbent body which can absorb urine. The device appears to be designed for single use when needed and immediate disposal thereafter.

Some other devices are designed to cover the whole male genitalia, i.e. penis and scrotum. These devices are naturally somewhat larger in dimension and therefore provide more storage capacity.

For example, DE 196 37851 discloses an incontinence device comprising a housing portion, which can be joined to a piece of underwear and can be reused. Provided therein is an absorbent member which is intended for disposal after use. This two piece device appears to be somewhat bulky.

WO 85/03428 discloses an incontinence device consisting of a protection bag having an opening for the introduction of the male genitalia. The bag comprises two walls which roughly have the form of parallelograms and which are attached to each other along three of the respective edges. It is difficult to see how this device can be worn inconspicuously under garments.

WO 96/20665 discloses another incontinence guard to be positioned around the male genitalia. The guard is provided in a specific form, its cross section having the form of a Z whose base is joined to a mirror image of that Z and provides an essentially V-shaped opening for insertion of the genitalia. The guard makes use of (non optional) fastener devices for fastening to a diaper, underpant or the like. It appears, while this form of an incontinence may allow relatively cost-effective production, that the V-shaped opening does not correspond to the form of the male genitalia in an ideal manner.

WO 99/33422 discloses an incontinence article for males comprising a pouch member. The article is so designed that the genitalia are to be inserted with the penis pointing upwardly. This position of the penis makes it likely that the genitalia come in contact with urine dripping down under the influence of gravity and further is this position by some users considered an unnatural and uncomfortable position.

Japanese utility model application Hei 10-6747 discloses an incontinence article comprising two sheets which are joined along two of their respective sides to form a pouch. The sheets are provided in the form of squares. Again, it appears difficult to see how the shape of this product corresponds to the male anatomy and provide good and comfortable fit - even when fastening means are used.

While the above described incontinence products comprise a large variety of different shapes and considerably efforts in this field have been directed to the optimisation of the device, there remains a need for providing a device suitable for daily use by active wearers, in particular those wearing modern western fashion, such as trousers and jeans.

In view of the prior art there remains a need for an incontinence device which:
- provides sufficient capacity for medium or severe urine incontinence
- can be worn inconspicuously, in particular under western style garments
- can be worn comfortably, in particular by an active wearer
- reliably stores liquid, also when used by an active wearer
- does not give a wet or clammy feeling in use
- engages in reliable seal with the human body
- provides protection from odours
- can easily be positioned and applied
- can readily and cheaply be produced

These and other objectives are addressed by the present invention as apparent from the following description.

### Summary of the invention

The present invention relates to an absorbent article, more particularly to an absorbent article to be worn by incontinent males. Claimed and described is an absorbent article being provided from a substantially rectangular sheet of material, the sheet of material comprising at least one line of juncture where the sheet is joined to itself as to form a pouch, characterised in that the sheet has a length to width ratio of at least 1.2:1. In another aspect of the present invention the absorbent article can be used in two different orientations.

### Brief description of the drawings

It is believed that the invention will be better understood from the foregoing description in conjunction with the accompanying drawings in which:

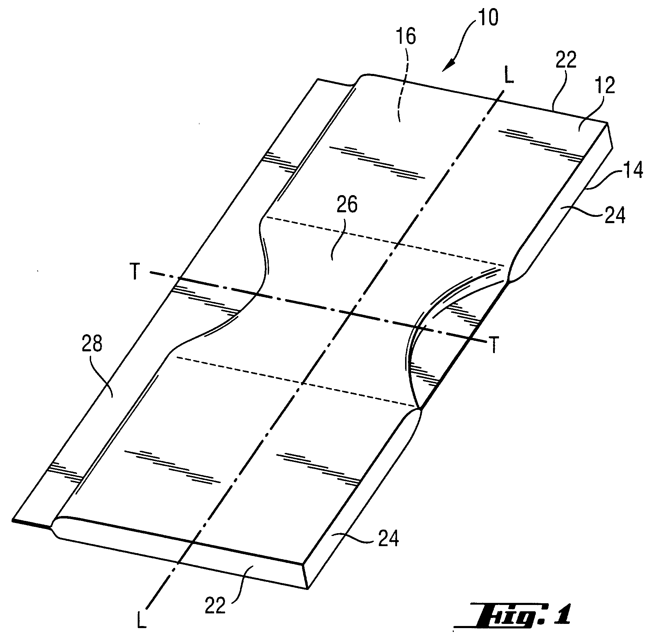

Figure 1 is a view of a sheet used to provide an absorbent article according to the present invention.
Figure 2 is a perspective view of the sheet of the present invention when folded and joined in a preferred way so as to form a pouch.
Figure 3 is a perspective view of the pouch as shown in Figure 2 as formed into a body-fitting configuration.
Figure 4 is a partially cut-away view, a preferred embodiment of the present invention as worn on the body.

### Detailed description of the invention

The present invention relates to a male incontinence device. According to the present invention the device comprises a sheet (10), preferably comprising fluid storage material. In a preferred embodiment this sheet (10) further comprises a liquid permeable layer as a wearer facing outer layer also referred to as topsheet (12), a liquid impermeable layer as a garment facing second outer layer also referred to as backsheet (14) and an absorbent core (16) disposed in-between. The sheet (10) will be discussed in more detail hereinafter.

According to the present invention the sheet is provided in a substantially rectangular form. A rectangular form has been found to provide best fit and comfort of the pouch formed by the sheet (10).

The term "rectangular", as used herein, with reference to the sheet (10), refers to sheets which can be encompassed by a rectangle whereby the areas between the outer contours of the sheet (10) and the sides of the rectangle in total measure less than 20%, preferably less than 15%, more preferably less than 10%, most preferably less than 5% of the total area of the rectangle. Therefore, for example, a sheet (10) with a slightly irregular contour or an hour-glass shaped sheet (10) are also referred to as rectangular herein. The term "rectangle encompassing the sheet (10)", as used herein, denotes the rectangles with encompasses the sheet (10) which is smallest in area.

According to the present invention the sheet (10) should have specific length to width ratios. As used herein the term "length" refers to the length of the longer sides of the rectangle encompassing the sheet (10) and the term "width" refers to length of the shorter sides of the rectangle encompassing the sheet (10). The terms "length" and "width", as used herein, refer to measures which define the form of the incontinence device. Therefore, portions of the sheet (10) which form an overlap when the sheet (10) is formed into the pouch of the present invention are not taken into account.

Preferably, the length to width ratio is from 1.2:1 to 10:1, preferably from 1.5:1 to 5.0:1, yet more preferably 1.8:1 to 4.0:1, yet more preferably 2.0:1 to 3.0:1, most preferably 2.5:1 to 2.8:1. Further the rectangle encompassing the sheet (10) preferably has an absolute width of 5 cm to 30 cm, more preferably 10 cm to 25 cm, most preferably 12 cm to 18 cm and a length of preferably 10 cm to 60 cm, more preferably 25 cm to 50 cm, most preferably 30 cm to 40 cm when the incontinence device is provided for an adult male.

While the sheet (10) in one preferred embodiment of the invention comprises only parts which extend over substantially the whole area of the sheet (10), in other embodiments of the present invention the sheet (10) may be provided by a number of separate pieces which are joined together by any means known in the art. Such means to join pieces namely include thermo-bonding, ultrasonic-bonding, adhesive means, stitching, thermo-crimping and cold-crimping. Alternatively, several pieces of a sheet (10), for example several pieces of the absorbent core, can be hold together by parts of the sheet (10) extending over the whole area of the sheet (10), for example the topsheet (12) and the backsheet (14).

According to the present invention a pouch is provided from the sheet (10) by joining at least one side of the sheet (10) to itself along at least one line of juncture. In a highly preferred embodiment of the present invention only one side of the sheet (10) is joined to itself so as to form a pouch, embodiments in which two sides are respectively joined to themselves are also preferred. As opposed to any others joints which may be comprised by the sheet (10), the sheet (10) without this at least one line of juncture is essentially flat, whereas it is turned into a pouch by this at least one line of juncture. This line of juncture can be provided by any means known in the art, as listed above, and may be continuous or intermittent. In one preferred embodiment of the present invention the rectangular sheet (10) is folded over itself about a central transversal line, more preferably the sheet (10) is folded about two lines which are parallel and equidistant to the central transversal axis and spaced apart 2 cm to 10 cm, preferably 6 cm. In a highly preferred embodiment one line of juncture is provided along one of the longitudinal sides.

"Longitudinal" as used herein, refers to the direction parallel to the longer side of the rectangle comprising the sheet (10). "Transversal" as used herein, refers to the direction parallel to the shorter side of the rectangle comprising the sheet (10).

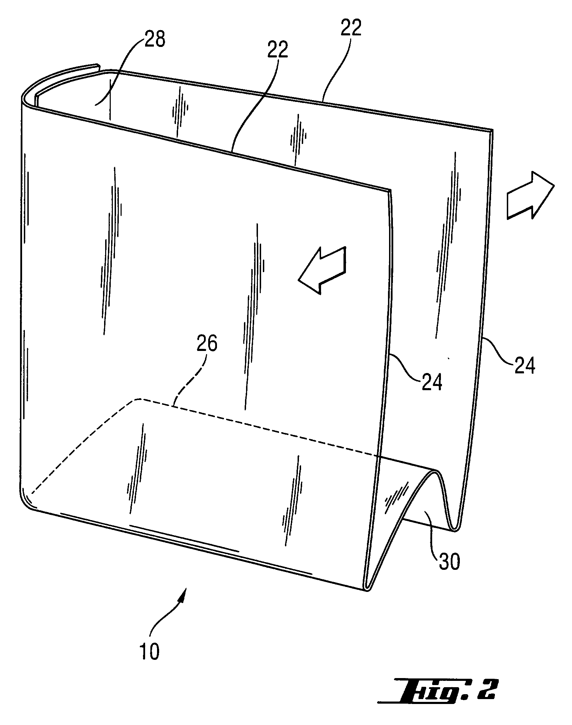

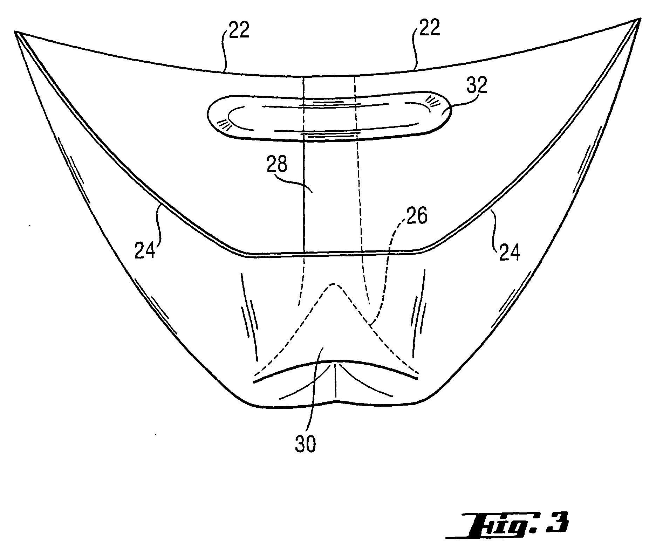

This preferred embodiment is shown in Figure 2. Due to the flexibility of the sheet material the form of the pouch shown in Figure 2 can readily be converted into the somewhat triangular form of the pouch shown in Figure 3 (as indicated by the two arrows in Figure 2). This overall triangular form of a pouch was found to provide high wearing comfort and fit.

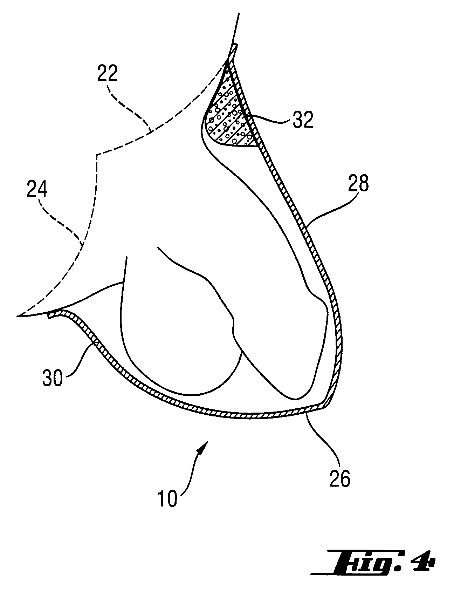

The pouch as shown in Figure 3 comprises a front edge (22) and rear edge (24) and a distal end (26). When the pouch is worn the front edge (22) has contact with the lower abdomen of the wearer above the penis whereas the rear edge (24) is in contact with the perineum and the scrotum of a wearer. The pouch comfortably provides space for both the scrotum and the penis of a wearer with the penis generally being oriented downwards and typically towards the distal end (26) - as shown in Figure 4.

It has been found that the rectangular form of the sheet (10) according to the present invention results in a pouch form, which in its wearing configuration shown in Figures 3 and 4 has a higher central front portion (28) than central rear portion (30). This means that length of the central front portion (28), measured as the length of a line perpendicular to the front edge (22) and reaching most distal point of the distal end (26), is greater than the length of the central rear portion (30), measured as the length of a line perpendicular to the rear edge (24) and reaching the most distal point of the distal end (26). Without wishing to be bound by theory, it is believed that the high wearing comfort provided by the present absorbent article is to a considerable extent due to this asymmetry in length. Preferably the length of the central front portion (28) is at least 10%, more preferably 20%, yet more preferably 30%, most preferably 40% greater than the length of the central rear portion (30).

In a further aspect of the present invention it has been found that the present design of an absorbent article, which in a preferred embodiment is a male incontinence article, allows to use the article in at least two different usage positions. While the usage position shown in Figure 4, where the front edge (22) is in contact with the wearer's abdomen, is preferred for use by an active wearer and when the article is worn under jeans or trousers, an alternative usage position can be chosen e.g. for bedridden wearers. In this alternative usage position, herein referred to as "night position", the rear edge (24) is in contact with the wearer's abdomen and the front edge (22) is in contact with the wearer's scrotum. Thereby the longer central front portion (28) underlies the scrotum.

This usage position is beneficial for a lying wearer, where leakage most likely occurs in the area of the scrotum, but by the present invention is prevented by providing ample absorbent material in this critical area. When worn in the night position the article is potentially more conspicuous under tight fitting garments, however, the product is then typically worn under night wear so that this is not a concern.

Hence the present invention discloses a male incontinence article which can be worn in a usage position which can be selected from at least two usage positions with substantially different orientations. The orientation of the article can be defined by using any axis which is fixed with regard to the article as a reference axis. The term substantially different orientation, as used herein, refers to a second orientation in which said axis is tilted as compared to the position of the axis in a first orientation by an angle of at least 30°, more preferably at least 90°, yet more preferably at least 160°, most preferably around 180°.

In a preferred embodiment the article comprises at least one foam pad (32) for additional protection against leakage, both by better seal to the body of the wearer and provision of additional absorbent capacity in critical areas. One preferred position for such foam pad is on the wearer facing side of the central front region (28) close to the front edge (22). Depending on the particular embodiment of the present invention the foam is best chosen to be hydrophobic or hydrophilic. In addition or as an alternative to such a foam pad (32) the front edge (22) and/or the rear edge (24) may be provided with a barrier means, preferably an elasticated barrier means.

In a highly preferred embodiment of the present invention the central front portion (28) running from the front edge (22) to the distal end (26) - at least partially - is not provided with a fluid storage material as defined hereinafter. Either several separate areas free of a fluid storage material or preferably one continuous area is free of a fluid storage material. Preferably on the wearer facing side the total surface of the area free of a fluid storage material measures at least 10 cm², more preferably at least 25 cm², yet more preferably at least 40 cm² and one continuous area without fluid storage material is comprised which has a surface area on the wearer facing side of at least 10 cm², preferably at least 25 cm², yet more preferably at least 40 cm². It has been found that absence of a fluid storage material in this area allows to provide a less bulky product. Further the chance of unpleasant re-wet from the fluid storage material in this area avoided. In fact, this central area is subject to most pressure, potentially causing re-wet from the fluid storage material since this central area is somewhat protruding. Further this central area has most contact with the male genitalia which makes re-wet in this area most unpleasant. Sufficient fluid storage material can be provided to the left and to the right of this central area, thereby essentially in the groin area of the wearer, and further underneath the scrotum. Typical western fashion, namely trousers and jeans, provides sufficient space in the groin area for fluid storage material to be worn inconspicuously, whereas the provision of fluid storage material in the central area make the product more conspicuous. Similarly, fluid storage material can be inconspicuously provided underneath the scrotum.

In one preferred embodiment of the present invention the front edge (22) and the rear edge (24) of the pouch are provided with a body compatible adhesive to ensure an even better seal to the body.

The form of the pouch comprising a rectangular sheet (10) of the material does not only provide great benefits for the comfort and functioning of the product but also allows cost-effective manufacture of the product. A variety of absorbent products is known which make use of essentially rectangular sheets of absorbent material, for example sanitary napkins or diapers. There is high production capacity in the respective industry to readily provide sheets as required by the present invention. Therefore, without making many adaptations to known production processes an absorbent incontinence article according to the present invention can be provided from such rectangular sheets.

### The absorbent sheet component

The sheet (10) preferably comprises a topsheet (12), a backsheet (14), an absorbent core (16), preferably comprising fluid storage material, and has longitudinal side margins and transversal side margins.

### The topsheet

The topsheet (12) is compliant, soft feeling, and non-irritating to the wearer's skin. The topsheet (12) also can have elastic characteristics allowing it to be stretched in one or two directions in portions of the topsheet (12) or throughout its extension. Further, the topsheet (12) is fluid pervious permitting fluids (e.g. urine) to readily penetrate through its thickness. A suitable topsheet (12) can be manufactured from a wide range of materials such as woven and non woven materials; polymeric materials such as apertured formed thermoplastic films, apertured plastic films, and hydroformed thermoplastic films; and thermoplastic scrims. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in US 3,929,135; US 4,324,246; US 4,342,314; US 4,463,045; and US 5,006,394. Particularly preferred micro apertured formed film topsheets are disclosed in US 4,609,518 and US 4,629,643. A preferred topsheet (12) for the present invention comprises the formed film described in one or more of the above patents and marketed on sanitary napkins by The Procter & Gamble Company of Cincinnati, Ohio as "DRI-WEAVE".

Topsheets not having a homogeneous distribution of liquid passage ways but only a portion of the topsheet (12) comprising liquid passage ways are also contemplated by the present invention. Typically such topsheets would have the liquid passage ways oriented such that they result in a centrally permeable and peripherally impermeable topsheet (12) for liquids.

The body surface of the formed film topsheet (12) can be hydrophilic so as to help liquid to transfer though the topsheet (12) faster than if the body surface was not hydrophilic. In a preferred embodiment, surfactant is incorporated into the polymeric materials of the formed film topsheet (12) such as is described in PCT-publication WO 93/09741. Alternatively, the body surface of the topsheet (12) can be made hydrophilic by treating it with a surfactant such as is described in US 4,950,254.

Another alternative are so called hybrid topsheets which incorporate fibrous and film like structures particularly useful embodiments of such hybrid topsheets are disclosed in PCT publications WO 93/09744; WO 93/11725 or WO 93/11726.

The topsheet (12) typically extends across the whole of the absorbent structure and outside the area coextensive with the absorbent structure. The topsheet (12) can extend and form part or all of the preferred side flaps.

When referring to the topsheet (12) a multi layer structure or a mono layer structure is contemplated. The hybrid topsheet (12) mentioned above is such a multi layer design but other multi layer topsheets such as primary and secondary topsheet (12) designs are also considered.

### Absorbent core

According to the present invention the absorbent cores suitable for use in herein may be selected from any of the absorbent cores or core system known in the art. As used herein the term absorbent core (16) refers to any material or multiple material layers whose primary function is to absorb, store and distribute fluid.

According to the present invention, the absorbent core (16) can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a. Primary/Secondary Fluid Distribution Layer

One optional component of the absorbent core (16) according to the present invention is a primary fluid distribution layer and a secondary fluid distribution layer. The primary distribution layer typically underlies the topsheet (12) and is in fluid communication therewith. The topsheet (12) transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b. Fluid Storage Layer

Positioned in fluid communication with, and typically underlying the primary or secondary distribution layers, is a fluid storage layer which comprises fluid storage material. The term "fluid storage material", as used herein, denotes any material with a absorbent capacity of more than 1g, preferably 2g, more preferably 3g of destilled water per g of fluid storage material, preferred fluid storage materials are hydrogels, superabsorbent material and hydrocolloid materials. Typically these materials are used in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water. Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c. Optional Fibrous ("Dusting") Layer

An optional component for inclusion in the absorbent core (16) according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core (16). Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d. Other Optional Components of the absorbent structure

The absorbent core (16) according to the present invention can include other optional components normally present in absorbent webs. For example, a reinforcing scrim can be positioned within the respective layers, or between the respective layers, of the absorbent core (16) . Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core (16) according to the invention and preferably is provided close to or as part off the primary or secondary fluid distribution layer are odour control agents.

### Backsheet

The backsheet (14) primarily prevents the extrudes absorbed and contained in the absorbent structure from wetting articles that contact the absorbent product such as underpants, pants, pyjamas and undergarments. The backsheet (14) is preferably impervious to liquids (e.g. urine) and is preferably manufactured from a thin plastic film, although other flexible liquid impervious materials can also be used. As used herein, the term "flexible" refers to materials that are compliant and will readily conform to the general shape and contours of the human body. The backsheet (14) also can have elastic characteristics allowing it to stretch in one or two directions.

The backsheet (14) typically extends across the whole of the absorbent structure and can extend into and form part of or all of the preferred sideflaps, side wrapping elements or wings.

The backsheet (14) can comprise a woven or nonwoven material, polymeric films such as thermoplastic films of polyethylene or polypropylene, or composite materials such as a film-coated nonwoven material. Preferably, the backsheet (14) is a polyethylene film having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet (14) is preferably embossed and/or matt finished to provide a more clothlike appearance.

Further, the backsheet (14) can permit vapours to escape from the absorbent structure, i.e. be breathable, while still preventing extrudates from passing through the backsheet. Also breathable backsheets comprising several layers, e.g. film plus non-woven structures, can be used. Such backsheets thus comprise at least one gas permeable layer. Suitable gas permeable layers include 2-dimensional, planar micro and macro-porous films, macroscopically expanded films, formed apertured films and monolithic films. The apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional planar layers of the backsheet (14) may be made of any material known in the art, but are preferably manufactured from commonly available polymeric materials. Suitable materials are for example Gortex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1 mm, preferably less than 0.5 mm, wherein the apertures have an average uniform diameter along their length and which do not protrude out of the plane of the layer. The apertured materials for use as a backsheet (14) in the present invention may be produced using any of the methods known in the art such as described in EPO 293 482 and the references therein. In addition the dimensions of the apertures produced by this method may be increased by applying a force across the plane of the backsheet (14) layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3,929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4,637,819 and US 4,591,523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane™ available from B.F. Goodrich (Belgium).

Particularly preferred backsheets for the present invention comprise at least two layers comprising at least one layer selected from the above, such as microporous and apertured formed films and an additional layer which may also be selected from the above listed backsheets or may be a fibrous woven or nonwoven. The most preferred breathable backsheet (14) component comprises a microporous film and an apertured formed film or a microporous and a hydrophobic woven or nonwoven material.

The absorbent article preferably also has fasteners for securing the absorbent pad in place in a wearer's undergarment or incontinence garment. The fasteners used with the absorbent pad are not limited to adhesive fasteners. Any suitable type of fastener known in the art can be used for this purpose. For example, the absorbent pad could be secured in place in a wearer's undergarment by mechanical fasteners, such as VELCRO™ or by a combination of adhesive and mechanical fasteners. For simplicity, however, the fasteners will be described in terms of adhesive fasteners and these fasteners are preferably pressure sensitive adhesive fasteners. Suitable pressure sensitive adhesive fasteners are described in greater detail in US 4,917,697.

## Claims

1. An absorbent article being provided from a substantially rectangular sheet (10) of material, said sheet (10) of material having four sides, a length and a width, said sheet (10) of material further comprising at least one line of juncture where at least one of said sides of said sheet (10) is joined to itself so as to form a pouch, **characterised in that** said ratio of said length to said width is at least 1.2:1.

2. An absorbent article according to Claim 1, **characterised in that** said ratio is at least 2:1.

3. An absorbent article according to any one of the preceding claims, **characterised in that** said sheet (10) comprises at least two layers of material.

4. An absorbent article according to any one of the preceding claims, **characterised in that** at least one of said layers comprises a fluid storage material.

5. An absorbent article according to Claim 4, **characterised in that** at least one portion of said sheet (10) is free said fluid storage material.

6. An absorbent article according to Claim 5, **characterised in that** one of said portions free of said fluid storage material comprises said line of juncture.

7. An absorbent article according to Claim 5, **characterised in that** one of said portions free of said fluid storage material has on the wearer facing side a total surface area of at least 25 cm2.

8. An absorbent article according to any one of the preceding claims, **characterised in that** said absorbent article is a male incontinence device.

9. Use of a male incontinence article in a usage position which can be selected from at least two usage positions with substantially different orientations.
